# EUROPEAN PATENT APPLICATION

(11) **EP 4 759 832 A1**
(43) Date of publication of application: **17.06.2026**
(21) Application number: 24218579.1
(22) Date of filing: 10.12.2024
(51) Int. Cl.: C07K 16/40, C07K 16/30, A61K 39/00, A61P 35/00

(54) **ANTIGEN-BINDING POLYPEPTIDES AGAINST CD203A**

(71) Applicant: Universitätsklinikum Hamburg-Eppendorf, 20246 Hamburg (DE)
(72) Inventor: Nolte, Friedrich, 20246 Hamburg (DE); Hambach, Julia, 20246 Hamburg (DE); Menzel, Stephan, 20246 Hamburg (DE); Haag, Friedrich, 20246 Hamburg (DE); Winzer, Riekje, 20246 Hamburg (DE); Tolosa, Eva, 20246 Hamburg (DE); Lorenz, Hannah, 20246 Hamburg (DE); Mann, Anna Marei, 20246 Hamburg (DE); Eggers, Marie, 20246 Hamburg (DE)
(74) Representative: Eisenführ Speiser

(57) **Abstract**

The invention relates to antigen-binding polypeptides comprising the CDR1, CDR2 and CDR3 region of a VHH domain of a camelid heavy chain antibody. The polypeptides bind specifically to CD203a and are therefore suitable for immune diagnostic and for immunotherapy that includes the targeting ofCD203a expressing cells, including tumor cells. In particular, the polypeptides are suitable for the therapeutic and prophylactic treatment of certain diseases that benefit for interfering with the enzymatic activity of CD203a. In addition, the polypeptides are suitable for the diagnosis as well as for the therapeutic and prophylactic treatment of diseases characterised by increased CD203a expression. Conjugates, viral vectors and pharmaceutical compositions comprising the antigen-binding polypeptides are also disclosed. In addition, the invention relates to the use of such antigen-binding polypeptides in methods for detecting CD203a or CD203a-expressing cells in a biological sample. Methods for purifying and concentrating CD203a or CD203a-expressing cells using the antigen-binding polypeptides are also described.

## Description

The invention relates to antigen-binding polypeptides comprising the CDR1, CDR2 and CDR3 region of a VHH domain of a camelid heavy chain antibody. The polypeptides bind specifically to CD203a and are therefore suitable for immune diagnostic and for immunotherapy that includes the targeting ofCD203a expressing cells, including tumor cells. In particular, the polypeptides are suitable for the therapeutic and prophylactic treatment of certain diseases that benefit for interfering with the enzymatic activity of CD203a. In addition, the polypeptides are suitable for the diagnosis as well as for the therapeutic and prophylactic treatment of diseases characterised by increased CD203a expression. Conjugates, viral vectors and pharmaceutical compositions comprising the antigen-binding polypeptides are also disclosed. In addition, the invention relates to the use of such antigen-binding polypeptides in methods for detecting CD203a or CD203a-expressing cells in a biological sample. Methods for purifying and concentrating CD203a or CD203a-expressing cells using the antigen-binding polypeptides are also described.

### BACKGROUND

The search for new therapeutic targets has led to the identification of immunosuppressive adenosine as an important regulator of anti-tumour immunity. Selective inhibitors targeting various components of the adenosinergic pathway have been contemplated for use in cancer therapy. Components of the adenosinergic pathway which are of particular interest in this respect include CD203a.

CD203a (ENPP1) is a cell surface ectoenzyme that catalyses the hydrolysation of adenosine triphosphate (ATP), cyclic guanosine monophosphate-adenosine monophosphate (cGAMP), and adenosine bisphosphate ribose (ADPR) to adenosine monophosphate and other products. The human protein is predicted to consist of 925 amino acids. CD203a exists as a homodimer of which each monomer is characterized by a short amino-terminal intracellular portion, a single transmembrane domain and a large extracellular domain. Expression of CD203a has been detected in almost all tissues. In the murine immune system CD203a expression is especially high in plasma B cells. In addition, high levels of CD203a and ectopic expression have been reported for various types of cancers. High expression levels of CD203a are expected to have direct effects on the immune system, as infiltration and function of immune cells is hampered.

In addition, tumour growth has been reported to be enhanced. This suggests that CD203a is involved in the regulation of the immune response and the development and progression of various types of cancer.

Accordingly, CD203a represents an interesting target for the diagnosis and therapy of various cancer diseases. There are small molecules and monoclonal antibodies against CD203a, which can be used in the context of diagnostics and therapy. However, the treatment of tumours with monoclonal antibodies is regularly associated with considerable disadvantages. For example, monoclonal antibodies often show an insufficient stability after administration into a patient. In addition, the costs involved in the production of monoclonal antibodies are very high. Specifically, the production of humanised antibodies with decreased immunogenicity is laborious and cost intensive. In addition, owing to their size, complete antibodies show limited tissue permeability.

Various strategies have been developed to solve the problems associated with the administration of antibodies. For example, fragments of full IgG antibodies are discussed for therapeutic purposes. However, these fragments often show reduced specificity and affinity with respect to the antigen compared to the complete antibody. Furthermore, these fragments often show only low solubility in aqueous solutions.

It has been described that camelid antibodies have properties that render them particularly suitable for therapeutic use in humans. In addition to conventional antibodies, which each have two heavy and two light chains, camelids also produce antibodies that exclusively consist of heavy chains. These so-called heavy chain antibodies are homodimers of two identical heavy chains that interact with the antigen through a single variable domain called VHH (variable domain of the heavy chain of heavy chain antibodies).

The VHH domain of a camelid heavy chain antibody has three complementary determining regions (CDRs) and four framework regions (FRs) that alternate with the CDRs in the primary sequence of the VHH domain. The VHH domain of a heavy chain antibody hence forms a small polypeptide unit with high antigen-binding capacity. Due to an exceptionally long CDR3 region with a length of 7-25 amino acids, the VHH domain can bind in cavities of protein antigens, unlike conventional antibodies, and thus block the active site of an enzyme. VHH domains can be produced recombinantly as soluble proteins in bacteria or mammalian cells. Such recombinantly produced VHH domains are also called single-domain antibodies (sdAbs) or nanobodies. Currently, several nanobodies are being tested in phase II clinical trials.

VHH single domain antibodies are more stable and about ten times smaller than conventional antibodies and therefore exert a high solubility and significantly improved tissue penetration properties. For this reason, VHH single domain antibodies have been proposed for the treatment of various diseases.

The present invention provides for the first time VHH single domain antibodies capable of specifically binding to CD203a. Such VHH single domain antibodies directed against CD203a have not been known in the prior art. The single domain antibodies of the present invention are particularly suitable for immunotherapy that includes the modulation, and particularly the blocking, of CD203a function and the targeting of CD203a expressing cells. In particular, the polypeptides are suitable for the therapeutic and prophylactic treatment of certain diseases that benefit from interfering with the function, such as the enzymatic activity, of CD203 a that occurs on cells, on microvesicles or in soluble form. In addition, the polypeptides are suitable for use in diagnosis and the therapeutic treatment of diseases characterised by increased expression of CD203a.

### DESCRIPTION OF THE INVENTION

In the present invention, heavy chain antibodies were first generated by immunising alpacas with human or murine CD203a. Subsequently, RNA was isolated from peripheral blood lymphocytes of the alpacas and converted into cDNA by reverse transcription. Based on the isolated RNA, the variable domains of the heavy chain antibodies were amplified by PCR. VHH genes were cloned into a eukaryotic expression vector, recombinantly expressed in HEK-6E cells and screened for CD203 binding on transfected CHO cells. Hits were sequenced, re-cloned to different formats, produced and purified for further analyses. In this way, the present invention provides VHH single domain antibodies suitable for use in therapeutic and diagnostic methods. In addition, the present invention provides CDR regions from VHH single domain antibodies that can be used in the construction of recombinant antigen-binding polypeptides.

Accordingly, in a first aspect, the invention provides an antigen-binding polypeptide comprising the complementarity-determining regions CDR1, CDR2 and CDR3 of a VHH domain of a camelid heavy chain antibody necessary for binding of a VHH single domain antibody, wherein the polypeptide specifically binds to CD203a.

The term "CD203a" as used herein refers to the "cluster of differentiation protein 203a". In a preferred embodiment, the protein to which the polypeptide of the invention binds is human CD203a (gene ID 5167) which is referred to herein as SEQ ID NO:173. In a particularly preferred embodiment, the polypeptide of the invention specifically binds to an epitope that is located in the extracellular domain, more preferably in the conserved alkaline phosphodiesterase I domain or in the nucleotide pyrophosphatase domain of the extracellular domain. The extracellular domain of CD203a extends from amino acid 98 to amino acid 925.

The polypeptides according to the invention comprise CDR regions derived from heavy chain antibodies of camelids. Heavy chain antibodies are a particular group of immunoglobulins consisting exclusively of heavy chains. In addition to two constant domains, a heavy chain of such an antibody contains a single variable domain, which in turn consists of three CDR regions and four FR regions. Interaction with the antigen takes place exclusively via this variable domain, which is also referred to as the VHH domain. Due to distinctive loop structures in the CDR3, a VHH domain is also able to recognise cryptic epitopes. Heavy chain antibodies are produced in only a few species, e.g. in llamas and alpacas. The camelid group is a family of mammals from the order Artiodactyla (even-toed ungulates). The camelid family includes camels, dromedaries, llamas, alpacas, guanacos and vicuñas. In a particularly preferred embodiment of the invention, the antigen-binding polypeptide comprises CDR regions derived from a VHH domain of a llama or alpaca, and more preferably an alpaca.

The polypeptide according to the invention has a specific affinity for CD203a, and more preferably human CD203a. This means that a polypeptide according to the invention binds to CD203a with a binding affinity that is significantly higher than the binding affinity for binding to another protein that is not homologous to the sequence of CD203a.

Preferably, the binding affinity of the polypeptide according to the invention is at least 2-fold, at least 5-fold, at least 10-fold, at least 20-fold, at least 50-fold, at least 75-fold, at least 100-fold, at least 150-fold, at least 200-fold, at least 250-fold, at least 500-fold, at least 600-fold, at least 700-fold, at least 800-fold, at least 900-fold, at least 1000-fold, at least 2000-fold, at least 5000-fold, or at least 10000-fold higher than the binding affinity for a protein not homologous to CD203a, such as human albumin.

The binding affinity of the polypeptide according to the invention will preferably bind to its antigen with a dissociation constant (Kd) of 10⁻⁵ M or less, and more preferably in the range of 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. Accordingly, the polypeptide according to the invention will preferably bind to its antigen with a dissociation constant (Kd) of 10⁻⁵ M to 10⁻¹² M, and more preferably 10⁻⁶ M to 10⁻¹² M, 10⁻⁷ M to 10⁻¹² M, 10⁻⁸ M to 10⁻¹² M, or 10⁻⁹ M to 10⁻¹² M. A Kd value of greater than 10⁻⁴ M is generally considered to indicate non-specific binding. Methods for measuring the binding affinities of antibodies are well known in the prior art and include, for example, the determination of Kd values by flow cytometry, surface plasmon resonance spectroscopy or microscale thermophoresis.

In another preferred embodiment, the polypeptide according to the invention will bind to CD203a, and more preferably to human CD203a, and modulate its function, e.g., reduce or block its enzymatic activity, i.e. the ectonucleotide pyrophosphatase/phosphodiesterase activity. Preferably, the enzymatic activity of CD203a, and more preferably human CD203a, is modified, and preferably reduced, by at least 50%, and more preferably by at least 60%, at least 70%, at least 80%, at least 90%, or at least 95%. Most preferably, the enzymatic activity of CD203a, and more preferably human CD203a, is either completely blocked or enhanced.

In a preferred embodiment of the invention, the CD203a-specific antigen-binding polypeptide comprises
(a) the CDR sequences shown in SEQ ID NO:1-3,
(b) the CDR sequences shown in SEQ ID NO:4-6;
(c) the CDR sequences shown in SEQ ID NO:7-9;
(d) the CDR sequences shown in SEQ ID NO:10-12;
(e) the CDR sequences shown in SEQ ID NO:13-15;
(f) the CDR sequences shown in SEQ ID NO:16-18;
(g) the CDR sequences shown in SEQ ID NO:19-21;
(h) the CDR sequences shown in SEQ ID NO:22-24;
(i) the CDR sequences shown in SEQ ID NO:25-27;
(j) the CDR sequences shown in SEQ ID NO:28-30;
(k) the CDR sequences shown in SEQ ID NO:31-33;
(l) the CDR sequences shown in SEQ ID NO:34-36;
(m) the CDR sequences shown in SEQ ID NO:37-39;
(n) the CDR sequences shown in SEQ ID NO:40-42;
(o) the CDR sequences shown in SEQ ID NO:43-45; or
(p) the CDR sequences shown in SEQ ID NO:46-48;
or CDR sequences which differ from the CDR sequences shown in (a)-(p) in not more than one amino acid per CDR region.

In another preferred embodiment of the invention, the CD203a-specific antigen-binding polypeptide comprises
(a') the CDR sequences shown in SEQ ID NO:49-51,
(b') the CDR sequences shown in SEQ ID NO:52-54;
(c') the CDR sequences shown in SEQ ID NO:55-57;
(d') the CDR sequences shown in SEQ ID NO:58-60;
(e') the CDR sequences shown in SEQ ID NO:61-63;
(f') the CDR sequences shown in SEQ ID NO:64-66;
(g') the CDR sequences shown in SEQ ID NO:67-69;
(h') the CDR sequences shown in SEQ ID NO:70-72;
(i') the CDR sequences shown in SEQ ID NO:73-75;
(j') the CDR sequences shown in SEQ ID NO:76-78;
(k') the CDR sequences shown in SEQ ID NO:79-81;
(l)' the CDR sequences shown in SEQ ID NO:82-84;
(m') the CDR sequences shown in SEQ ID NO:85-87;
(n') the CDR sequences shown in SEQ ID NO:88-90;
(o') the CDR sequences shown in SEQ ID NO:91-93;
(p') the CDR sequences shown in SEQ ID NO:94-96,
(q') the CDR sequences shown in SEQ ID NO:97-99;
(r') the CDR sequences shown in SEQ ID NO:100-102;
(s') the CDR sequences shown in SEQ ID NO:103-105;
(t') the CDR sequences shown in SEQ ID NO:106-108;
(u') the CDR sequences shown in SEQ ID NO:109-111;
(v') the CDR sequences shown in SEQ ID NO:112-114;
(w') the CDR sequences shown in SEQ ID NO:115-117;
(x') the CDR sequences shown in SEQ ID NO:118-120;
(y') the CDR sequences shown in SEQ ID NO:121-123;
(z') the CDR sequences shown in SEQ ID NO:124-126;
(ö') the CDR sequences shown in SEQ ID NO:127-129.
or CDR sequences which differ from the CDR sequences shown in (a')-(ö') in not more than one amino acid per CDR region.

With respect to the amino acid sequence set forth in SEQ ID NO:98 in the attached sequence listing, it is to be noted that only amino acids 1-3 (R-G-K) represent the CDR, while the amino acid T does not belong to the CDR.

In a particularly preferred embodiment of the invention, the CD203a-specific antigen-binding polypeptide comprises the CDR regions CDR1, CDR2 and CDR3 shown in SEQ ID NO:1-3.

In another particularly preferred embodiment, the CD203a-specific antigen-binding polypeptide comprises the CDR1 shown in SEQ ID NO:1, the CDR2 shown in SEQ ID NO:2, and the CDR3 selected from the group consisting of SEQ ID NO:3 and SEQ ID NO:6.

In another particularly preferred embodiment, the CD203a-specific antigen-binding polypeptide comprises the CDR1 shown in SEQ ID NO:10, the CDR2 shown in SEQ ID NO:11, and the CDR3 selected from the group consisting of SEQ ID NO: 12, SEQ ID NO:15, and SEQ ID NO:18. In another particularly preferred embodiment, the CD203a-specific antigen-binding polypeptide comprises the CDR1 shown in SEQ ID NO:34, the CDR2 shown in SEQ ID NO:35, and the CDR3 selected from the group consisting of SEQ ID NO:36 and SEQ ID NO:39.

It is apparent to the skilled person that within the presently disclosed CDRs of SEQ ID NO:1-48 and SEQ ID NO:49-129 some modification of the amino acid sequence is possible without affecting the binding ability of the VHH single domain antibody. For example, it may be possible to modify CDR1 of the single domain antibody by substituting 1, 2, or 3 amino acids. Similarly, it may be possible to modify CDR2 of the single domain antibody by substituting 1 or 2 amino acids. Due to the length of CDR3, it may be possible to modify this CDR by substituting 1, 2, 3, 4, 5, or 6 amino acids.

In general, any of the amino acid residues within the CDRs shown in SEQ ID NO: 1-48 and SEQ ID NO:49-129 may be replaced with another residue as long as the resulting CDR is still able (along with the other two CDRs) to specifically bind to CD203a. Furthermore, 1, 2 or 3 amino acids within one of the sequences shown in SEQ ID NO:1-48 and SEQ ID NO:49-129 may also be deleted or added by addition as long as the ability to bind to CD203a is not completely eliminated.

Thus, also encompassed by the invention are antigen-binding polypeptides having CDRs that differ from the sequences shown in SEQ ID NO:1-48 and SEQ ID NO:49-129 in individual amino acid positions. The invention therefore specifically relates to antigen-binding polypeptides having CDR sequences that differ from any of the CDR sequences shown in SEQ ID NO: 1-48 and SEQ ID NO:49-129 in not more than a single amino acid per CDR.

In a further preferred embodiment, the antigen-binding polypeptide of the invention comprises a complete VHH domain of a camelid heavy chain antibody. Such a VHH domain, when used alone, is capable of binding to the corresponding antigen and is therefore also referred to herein as a "VHH single domain antibody". In addition to the three CDR regions responsible for antigen binding, the VHH domain also comprises the four framework regions FR1-FR4, which are located between the CDR regions. The scaffold regions may be fully conserved in the VHH domain of the invention, or they may be in the form of fragments of the original scaffold sequences. For example, the scaffold sequences delimiting the VHH domain C-terminally and N-terminally may be truncated by one or more amino acids. Such modified VHH domains are expressly encompassed by the invention. As used herein, "VHH single domain antibody" means an antigen-binding polypeptide that lacks the constant domains CH2 or CH3 compared to a full heavy chain antibody. In a preferred embodiment, the antigen-binding polypeptide of the invention is a VHH single domain antibody.

Preferred antigen-binding polypeptides comprising a VHH domain of a camelid heavy chain antibody and specifically binding to CD203a comprise or consist of the sequences depicted in SEQ ID NO: 130-145 and SEQ ID NO: 146-172. The invention further extends to variants of the polypeptides shown in SEQ ID NO: 130-145 and SEQ ID NO: 146-172 that are homologous to the polypeptides shown in SEQ ID NO: 130-145 or and SEQ ID NO: 146-172 and also have the ability to specifically bind CD203a. As used herein, the term "variant" means an amino acid sequence that has been modified by deletion, substitution or addition of amino acids such that it has at least 80%, 85%, 90%, 91%, 92%, 93%, 94%, 95%, 96%, 97%, 98%, 99% sequence identity to any of the sequences shown in SEQ ID NO: 130-145 and SEQ ID NO: 146-172 when those sequences are optimally aligned, e.g., with the GAP or BESTFIT programs using the default gap procedure. Computer programs for determining amino acid sequence identity are sufficiently known in the prior art.

VHH single domain antibodies are distinguished from conventional mammalian antibodies in particular by the fact that only three CDRs are required for binding the antigen. The invention therefore also relates to those antigen-binding polypeptides which comprise only the CDR1, CDR2 and CDR3 regions required for binding CD203a of one of the polypeptides exemplified in SEQ ID NO: 130-145 and SEQ ID NO: 146-172, wherein the regions located between the CDR regions show only minor or essentially no similarity to the corresponding regions of one of the polypeptides shown in SEQ ID NO: 130-145 and SEQ ID NO: 146-172.

Thus, the framework regions (FR1-FR4) of the polypeptides provided herein can differ from that of the VHH single domain antibodies shown in SEQ ID NO: 130-145 and SEQ ID NO: 146-172 without adversely affecting the binding of the polypeptides. Preferably, the variants differ from the polypeptides shown in SEQ ID NO: 130-145 and SEQ ID NO: 146-172 only by the exchange of a few amino acids. Particularly preferably, the variants of the polypeptides shown in SEQ ID NO: 130-145 and SEQ ID NO: 146-172 differ from the polypeptides shown in SEQ ID NO: 130-145 and SEQ ID NO: 146-172 in less than 25, in less than 20, in less than 15, in less than 10, or in less than 5 amino acids. Preferably, variants of the polypeptides shown in SEQ ID NO: 130-145 and SEQ ID NO: 146-172 differ from the polypeptides shown in SEQ ID NO: 130-145 and SEQ ID NO: 146-172 in 5, 4, 3, 2 or only 1 amino acid position.

In one aspect, the invention thus relates to a CD203a-specific antigen-binding polypeptide comprising an amino acid sequence selected from the group consisting of:
(a) the amino acid sequences of SEQ ID NO: 130-145; and
(b) an amino acid sequence having at least 80% or more sequence identity to one of the amino acid sequences of SEQ ID NO: 130-145.

In another aspect, the invention thus relates to a CD203a-specific antigen-binding polypeptide comprising an amino acid sequence selected from the group consisting of:
(a) the amino acid sequences of SEQ ID NO:146-172 ; and
(b) an amino acid sequence having at least 80% or more sequence identity to one of the amino acid sequences of SEQ ID NO: 146-172.

Preferably, the substitutions that distinguish the variants from the VHH single domain antibodies shown in SEQ ID NO: 130-145 and SEQ ID NO: 146-172 are conservative substitutions, i.e. substitutions of one or more amino acid residues by an amino acid of similar polarity acting as a functional equivalent. Preferably, the amino acid residue serving as a substitution is selected from the same group of amino acids as the amino acid residue to be substituted. For example, a hydrophobic residue may be replaced by another hydrophobic residue, or a polar residue may be replaced with another polar residue having the same charge. Functionally homologous amino acids which may be used for conservative substitution include, for example, non-polar amino acids such as glycine, valine, alanine, isoleucine, leucine, methionine, proline, phenylalanine, and tryptophan. Examples of uncharged polar amino acids include serine, threonine, glutamine, asparagine, tyrosine, and cysteine. Examples of charged polar (acidic) amino acids include histidine, arginine, and lysine. Examples of charged polar (basic) amino acids include aspartic acid and glutamic acid.

Furthermore, variants of the sequences shown in SEQ ID NO: 130-145 and SEQ ID NO:146-172 also include polypeptides in which one or more amino acids have been inserted into the amino acid sequence of one of the polypeptides shown in SEQ ID NO: 130-145 and SEQ ID NO: 146-172. Such insertions can occur at any position of the polypeptides shown in SEQ ID NO: 130-145 and SEQ ID NO: 146-172, as long as the resulting variant of the polypeptide retains its immunological activity (i.e. the ability to specifically bind CD203a). Furthermore, proteins shown in SEQ ID NO:130-145 and SEQ ID NO:146-172 in which one or more amino acids within the sequence are missing compared to the reference polypeptide are also considered variants of the polypeptide shown herein. Such deletions may affect any amino acid positions within the sequences shown in SEQ ID NO: 130-145 and SEQ ID NO: 146-172. In particular, the deletions may be those involving two or more (e.g. 3, 4 or 5) consecutive amino acids from any of the sequences of SEQ ID NO: 130-145 and SEQ ID NO: 146-172. Preferably, fewer than 25, fewer than 20, fewer than 15, fewer than 10, fewer than 5 amino acid positions in total have been inserted or deleted in the variant compared to the respective reference polypeptide having one of the sequences shown in SEQ ID NO: 130-145 and SEQ ID NO: 146-172.

Also included in the invention are immunologically active fragments of the polypeptides (or variants thereof) shown in SEQ ID NO: 130-145 and SEQ ID NO: 146-172. Immunologically active fragments are understood herein to be sequences which differ from the amino acid sequences shown in SEQ ID NO:130-145 and SEQ ID NO:146-172 or variants thereof by the absence of one or more amino acids at the N-terminus and/or at the C-terminus of the protein and which are still capable of specifically binding to CD203a, preferably human CD203a. For example, immunologically active fragments of the sequence shown in SEQ ID NO:130-145 and SEQ ID NO:146-172 may be fragments lacking the first two N-terminal and/or C-terminal amino acids of the polypeptide shown in SEQ ID NO:130-145 and SEQ ID NO:146-172. The skilled person will readily be able to determine the ability of fragments to bind to CD203a, e.g. by competitive antibody assays.

The polypeptides shown in SEQ ID NO:130-145 and SEQ ID NO:146-172 and variants or fragments thereof may further be structurally modified at one or more positions in the amino acid sequence, such as by introducing a modified amino acid. According to the invention, these modified amino acids may be amino acids that have been modified by biotinylation, phosphorylation, glycosylation, acetylation, branching and/or cyclisation.

In some embodiments, it is preferred that the variants or fragments of the antigen-binding polypeptides of the invention retain more than 50% of the original binding affinity for CD203a. It is even more preferred that the variants or fragments retain more than 60%, 70%, 80%, 90%, 95%, 96%, 97%, 98% or 99% of the original binding affinity of the antigen-binding polypeptides of the invention, e.g. the polypeptides shown in SEQ ID NO:130-145 and SEQ ID NO:146-172. The binding affinity for CD203a can be measured using *in vitro* assays. In other embodiments, it may be preferred that the variants or fragments of the antigen-binding polypeptides of the invention have a lower binding affinity of less than 50% of the original binding affinity for CD203a. Such variants and fragments are preferably used in a multivalent targeting approach using e.g. a bispecific or multispecific polypeptide construct.

In another aspect, the invention provides a nucleic acid molecule encoding an antigen-binding polypeptide as defined above. The nucleic acid molecule may be DNA or RNA. Preferably, it is single-stranded or double-stranded DNA, such as genomic DNA or cDNA. Preferably, the polynucleotide encodes a polypeptide comprising one of the amino acid sequences shown in SEQ ID NO:130-145 and SEQ ID NO:146-172 or a variant of any of these.

The invention further comprises an expression vector comprising a nucleic acid molecule encoding an antigen-binding polypeptide of the present invention. Expression vectors are typically DNA constructs that have an origin of replication that allows the vector to replicate independently of the host cell. Expression vectors also include regulatory control elements that are functionally linked to the nucleotide sequences to be expressed and control their transcription and/or translation. Suitable control elements may be prokaryotic promoters (constitutive or regulatable, such as lac, trp, T3, T7 or gpt promoter), eukaryotic promoters (constitutive or regulatable, such as e.g. thymidine kinase promoter, SV40 promoter or CMV promoter), operators, transcription or translation enhancers, transcription or translation terminators, ribosome binding sites, polyadenylation signals, selection markers, silencers and repressor sequences. Numerous prokaryotic and eukaryotic expression vectors suitable for carrying out the present invention are known to those skilled in the art. Examples include the prokaryotic vectors pHEN2, pGEM, pBluescript and pUC, which are regularly used for heterologous expression in *E. coli.* Examples of eukaryotic expression vectors include pcDNA3.1, pOG44, pSV2CAT Rc/CMV, Rc/RSV, GEM1 and the like.

The invention also relates to a host cell comprising a nucleic acid molecule encoding an antigen-binding polypeptide of the present invention or an expression vector comprising such a nucleic acid molecule. In particular, it may be a prokaryotic or a eukaryotic host cell. Examples of host cells that may be used in the context of the invention include prokaryotic host cells, such as bacterial strains of *Escherichia coli, Bacillus subtilis* and the like. Suitable eukaryotic cells include yeast cells, insect cells, mammalian cells, plant cells and the like. The antigen-binding polypeptide of the present invention is preferably expressed in mammalian cells, e.g. HEK cells. The method of transforming or transfecting the host cell with an expression vector comprising a nucleic acid molecule encoding the antigen-binding polypeptide will depend on the type of expression vector and the host cell used. The skilled person will have no difficulty in selecting suitable vectors and host cells for recombinant expression.

The invention also provides a method for the recombinant production of an antigen-binding polypeptide of the invention, said method comprising: (a) culturing a host cell as described above under conditions that allow expression of a nucleic acid molecule encoding an antigen-binding polypeptide of the present invention; and (b) isolating the antigen-binding polypeptide from the culture. In the preparation of the antigen-binding polypeptides of the invention, the nucleic acid molecules, expression vectors and host cells described above may be used.

In another aspect, the invention relates to a method for detecting CD203a and/or CD203a-expressing cells in a biological sample, comprising the steps of
(a) contacting a CD203a-specific antigen-binding polypeptide described above and defined in the claims with the biological sample under conditions that allow the formation of complexes of the antigen-binding polypeptide and CD203a; and
(b) detecting the complexes of the polypeptide and CD203a/CD203a-expressing cells.

The method comprises, as a first step, the contacting of an antigen-binding polypeptide of the invention with the biological sample. This step is carried out under conditions that allow for the formation of binding complexes consisting of the antigen-binding polypeptide and CD203a and/or CD203a-expressing cells. Conditions that allow the formation of complexes consisting of the antigen-binding polypeptides of the invention and CD203a are known in the prior art and are sufficiently described in the context of binding of antibodies to protein antigens. Such conditions may include a temperature in the range of 4-37°C, a pH in the range of 6-8 and an incubation time in the range of several minutes to several hours. It will be apparent to one skilled in the art that suitable binding conditions for a given pair of an antigen-binding polypeptide and CD203a will depend on various factors, such as the concentration of the antigen-binding polypeptide, the anticipated concentration of CD203a in the sample, and the like. The skilled person will be able to determine suitable conditions for binding between polypeptide and CD203a based on routine experiments to optimise the formation of the binding complexes.

After the antigen-binding polypeptide has bound to CD203a and/or CD203a-expressing cells, the immune complexes consisting of CD203a or CD203a-expressing cells and the polypeptide specifically bound to them are detected. For this purpose, well-known methods can be used. For example, the antigen-binding polypeptide according to the invention can be provided with a detectable label prior to contacting the sample suspected of containing the CD203a and/or CD203a-expressing cells. The label may be, for example, a fluorescent, chemiluminescent or radioactive label. Labelling with enzymes, such as horseradish peroxidase or alkaline phosphatase, is also possible. Another method that allows detection of the complexes is the recombinant expression of the antigen-binding polypeptides as fusion proteins that have one or more peptide epitopes that can be visualised using labelled antibodies.

In a particularly preferred embodiment of the invention, the method is used for the quantitative detection of CD203a and/or CD203a-expressing cells in a biological sample. The quantitative detection can be carried out by methods that have been described in the prior art, for example by quantitative determination of a fluorescent, chemiluminescent or radioactive labelling of polypeptides in complexes. The determination can be carried out by comparison with a reference sample, i.e. by comparison with a sample containing a known amount of CD203a and/or CD203a-expressing cells.

In a further aspect, the invention relates to a method for purifying and/or concentrating CD203a and/or CD203a-expressing cells, comprising the steps of
(a) contacting a CD203a-specific antigen-binding polypeptide as described above and defined in the claims with the biological sample under conditions that allow the formation of complexes of the antigen-binding polypeptide and CD203a; and
(b) separating the complexes of the CD203a-specific antigen-binding polypeptide and CD203a/CD203a-expressing cells from the sample.

Preferably, the CD203a-specific polypeptide used in the above method is one comprising one or more of the CDR regions listed in SEQ ID NO:1-48 and SEQ ID NO:49-129 or modifications thereof as described above. In a particularly preferred embodiment, the CD203a-specific polypeptides comprise the sequences or fragments listed in SEQ ID NO:1-48 and SEQ ID NO:49-129 or immunologically active fragments or variants thereof as described above.

Purification and/or concentration of CD203a and/or the CD203a-expressing cells is preferably performed with antigen-binding polypeptides immobilised on a solid support, such as a material suitable for use in chromatographic separation procedures. Suitable column materials include Sepharose (e.g. Q-Sepharose, DEAE-Sepharose, SP-Sepharose) and the like. The CD203a-binding polypeptides may further be immobilised on beads, e.g., agarose or glass beads. Preferably, the beads are magnetisable which facilitates separation of the binding complexes adhering to the beads after incubation in CD203a-containing samples. Methods for coupling polypeptides to a solid support are sufficiently known in the prior art. For example, a coupling method may comprise the immobilisation of polypeptides which linked to biotin or derivatives of biotin to a support material that has been previously coated with streptavidin or derivatives thereof. Such compounds are commercially available from various suppliers. Alternatively, the protein may be directly bound to the support material by chemical reaction. The sample containing the CD203a and/or CD203a-expressing cells is passed over the support material so that the CD203a and/or CD203a-expressing cells are bound by the immobilised antigen-binding polypeptides. By using a suitable wash buffer, all non-specifically bound molecules and cells are washed from the support material while the CD203a and/or CD203a-expressing cells are retained by the support material. In a final step, CD203a can be eluted from the material using a suitable elution buffer (e.g. a high salt buffer), thus obtaining the purified and/or concentrated CD203a protein. Alternatively, the CD203a bound to the support material or the CD203a-expressing cells can be detected by an immunological detection method.

In some embodiments, the antigen-binding polypeptides are not coupled to a support. In these embodiments, separation from the sample can be achieved, e.g. by designing the CD203a-specific antigen-binding polypeptide of the invention as a fusion polypeptide that comprises an affinity tag. Such fusion polypeptides can be efficiently purified using a material that has a particularly high affinity for the affinity tag used. For example, the affinity tag may have 6-12 histidine residues that specifically interact with a chelating nickel ion matrix. Alternatively, the affinity tag may comprise a glutathione S-transferase that can be purified using a glutathione matrix. Numerous other affinity tags are known in the art and can be used in conjunction with the antigen-binding polypeptides of the invention. Examples of affinity tag and affinity ligand pairs include maltose-binding protein (MBP) and maltose; avidin and biotin; streptag and streptavin or neutravidin.

Fusion proteins having an affinity tag may be produced, for example, by ligation of a DNA encoding an antigen-binding polypeptide of the invention with a sequence encoding the affinity tag. In cases where the affinity tag is not a peptide, the affinity tag may be conjugated to the antigen-binding polypeptide by chemical coupling reactions. For example, the antigen-binding polypeptide may be chemically coupled to biotin. Labelling with biotin can also be achieved by attaching to the antigen-binding polypeptide a peptide that is recognised by a biotin-protein ligase (EC 6.3.4.15), such as BirA from *E. coli.* Suitable peptides include the 13 amino acid minimal biotinylation sequence of the biotin carboxy carrier protein (BCCP), the 15 amino acid variant thereof called AviTagTM, and the 76 amino acid BioEaseTM sequence, all of which are biotinylated by the biotin protein ligase BirA at a central lysine residue. Streptags such as Streptag II and One-STrEP-tag (IBA BioTAGnology, IBA GmbH, Germany) may also be used.

Furthermore, the antigen-binding polypeptides of the present invention can also be coupled with enzymes (such as alkaline phosphatase) with recognition sequences for enzymes (such as the above BirA biotinylation sequences) or other small molecules. Coupling of different antigen-binding polypeptides, such as VHHs directed against different epitopes of CD203a, is also possible to enhance the efficiency of binding to the target antigen. For example, two or more of the single domain VHH antibodies described above may be coupled to biotin via a BirA recognition sequence and subsequently bound to avidin or streptavidin, or to modified streptavidin such as neutravidin or streptactin, via the high affinity binding of biotin. Avidin and streptavidin are tetrameric molecules that can bind four molecules of D-biotin. Such multimerised, tetravalent VHH single domain antibodies have an increased affinity for CD203a. Streptavidin-coated beads, e.g. magnetic or Sepharose beads, can be used for multimerisation. Suitable beads are e.g. Strep-Tactin Magnetic Nanobeads (IBA BioTAGnology, IBA GmbH, Germany). The polypeptides of the present invention, and in particular the VHH single domain antibodies can of course also be coupled to immunoglobulins, e.g. to the Fc region of an immunoglobulin. In addition, the VHH single domain antibodies of the present invention can be prepared in the form of fusion proteins that comprise more than one VHH domain that binds to CD203a, such as 2, 3, 4 or 5 VHH domains, which are expressed as a single protein chain. The additional VHH domains of the fusion protein may bind to the same or to different epitopes of CD203a. Alternatively, the additional VHH domains of the fusion protein may bind to other target molecules, e.g. to CD38, CD39, CD73, CD4, CD8, CD19, CD20 and/or CD56, or to immune checkpoint proteins such as PD-1, PD-L1, CTLA-4, LAG3, TIM3, TIGIT, PVR-IG, PVR or PVR-L2. In a particular preferred embodiment, the invention relates to a fusion protein that comprises a first VHH directed against CD203a and a second VHH directed against CD73. In another preferred aspect, the invention relates to a conjugate construct that comprises a VHH directed against CD203a and a small molecule compound.

In a particularly preferred aspect of the invention, the CD203a-specific polypeptides described above, preferably those comprising one or more of the CDR regions listed in SEQ ID NO: 1-48 and SEQ ID NO:49-129 or modifications thereof as described above, and those comprising the sequences listed in SEQ ID NO: 1-48 and SEQ ID NO:49-129 or immunologically active fragments or variants thereof as described above, are used for therapeutic and diagnostic purposes.

The antigen-binding polypeptides of the present invention are particularly suitable for use in a method of diagnosing a disease characterised by increased expression of CD203a. The diagnosis is based on the detection of cells or tissues that express increased levels of CD203a. These cells and tissues can be detected via a suitable label after binding one of the polypeptides according to the invention.

Thus, the present invention also relates to a method of diagnosing a disease characterised by increased or decreased (such as the complete lack of) cellular expression of CD203a, said method comprising
(a) contacting a CD203a-specific antigen-binding polypeptide as described above and defined in the claims with a biological sample from a patient under conditions that allow the formation of complexes of the antigen-binding polypeptide and CD203a; and
(b) detecting the complexes of the polypeptide and CD203a/CD203a-expressing cells;
wherein detection of the complexes in step (b) indicates that the patient has a disease characterised by increased CD203a expression.

The disease to be diagnosed is preferably a hyperproliferative disease. It is particularly preferred that the disease is a solid tumour, such as gastric cancer, colon cancer, bladder cancer, glioma, glioblastoma, renal cancer, ovarian cancer, melanoma, prostate cancer, thyroid cancer, oesophageal cancer, lung cancer, such as non-small cell lung carcinoma (NSCLC), head and neck cancer, brain cancer, such as astrocytic brain tumors, breast cancer, and melanoma. In other embodiments, the disease is a haematological cancer disease, such as Burkitt's lymphoma, T-cell lymphoma, hairy cell leukaemia, chronic lymphocytic leukaemia (CLL), multiple myeloma, chronic myeloid leukaemia (CML), acute myeloid leukaemia (AML), and acute lymphoblastic leukaemia (ALL). In a particularly preferred embodiment of the invention, the method is used to diagnose a malignant solid tumor.

The invention may also serve as a diagnostic tool for detecting CD203a deficiency and as a monitoring system for evaluating the efficacy of CD203a supplementation therapy. Thus, in one aspect, the invention pertains to a method of detecting CD203a deficiency in a cell sample of a person, comprising
(a) providing a sample of CD203a-expressing cells from a test subject;
(b) detecting the level of CD203a expression on the surface of the cells;
(c) comparing the level of CD203a expression with the level of expression in a cell sample from a healthy subject,
wherein a reduction of CD203a expression in the sample from the test subject indicates that the test subject suffers from CD203a deficiency

The biological sample may be a tissue sample from a patient, e.g. a tissue sample from a tumour. It may also be a sample of a body fluid, such as blood or lymph, or a bone marrow sample. For example, the sample may be a blood sample containing B lymphocytes and/or T lymphocytes or other immune cells. Depending on the disease to be diagnosed, it may also be a specific fraction of a body fluid.

The number of complexes detected in step (b) may be compared in the course of diagnosis with a reference sample analysed under identical conditions. The reference sample may be from a patient who has already been diagnosed with the disease to be detected, e.g. one of the aboverecited cancers, such as multiple myeloma, CLL, CML, AML or ALL. In this case, a measurement indicating an approximately identical number of complexes in the reference sample and the patient sample indicates that the patient is also likely to be afflicted with the disease. Alternatively, the reference sample may be a sample from a healthy individual. In this case, the disease can be detected in the patient if the measured number of complexes in the patient sample is significantly higher compared to the reference sample.

In another embodiment, the present invention relates to a method for visualizing CD203a-expressing cells or tissues in a subject, comprising the step of
(a) administering to the subject a CD203a-specific antigen-binding polypeptide as described above and defined in the claims, wherein the polypeptide is provided with a detectable label; and
(b) visualizing the CD203a-expressing cells or tissues in the body by detecting the label.

The above method enables a so-called in vivo imaging of CD203a-expressing cells or tissue in a subject, such as a human. The polypeptides administered to the subject are provided with a label that allows detection of the label without explanation of the CD203a-expressing cells or tissues. Preferably, the label is a fluorescent label, such as an Alexa dye. Suitable Alexa dyes include, but are not limited to, Alexa-647 and Alexa-680. The amount of labelled polypeptide required for visualisation depends on various factors, such as the label as well as the weight of the subject to whom the polypeptide is administered. Quantities can generally be used as described below in the context of therapeutic treatment.

The CD203a-specific antigen-binding polypeptide described hereinabove can also be used therapeutically to treat diseases or disorders which can be ameliorated by modulating, such as blocking, reducing or increasing, the enzymatic activity of CD203a. Extracellular adenine nucleotides (AN) act as signal molecules that have been reported to be involved in regulating universal cellular processes, including cell-cell communication and intracellular signalling. In particular, AN appear to play an important role in mediating pro-inflammatory responses. Time and location of their release directly depends on the expression of enzymes involved in their conversion. Nicotinamide adenine dinucleotide (NAD) plays a significant role in the regulation of immune response. Extracellular NAD serves as a substrate for the enzyme CD38 which metabolizes NAD to ADP-ribose (ADPR). ADPR is further hydrolysed to adenosine monophosphate (AMP) and nucleoside adenosine (ADO) by CD203a and CD73. ATP can also be degraded by CD203a to AMP and further degraded by CD73 to ADO. ADO has been reported to have immunosuppressive effects. Signalling by these AN appears to be involved in immune responses against pathogens, autoimmunity and inflammatory reactions such as contact dermatitis, glomerulonephritis, multiple sclerosis, graft versus host disease, neuropathic pain and febrile response. Accordingly, blocking of CD203a and CD73 constitutes a reasonable approach for activating the immune response and overcoming immunosuppression in cancer diseases and chronic infections. In addition, blocking of CD203a and CD73 is useful for enhancing an immune response in a subject after vaccination.

Thus, another aspect of the present invention pertains to the antigen-binding polypeptides of the present invention for use in a method of treating a disease or disorder which is characterized by a pathologically enhanced level of CD203a activity. In a preferred embodiment, the disease to be treated is a solid tumour, such as gastric cancer, colon cancer, bladder cancer, glioma, glioblastoma, renal cancer, ovarian cancer, melanoma, prostate cancer, thyroid cancer, oesophageal cancer, lung cancer, such as non-small cell lung carcinoma (NSCLC), head and neck cancer, brain cancer, such as astrocytic brain tumors, breast cancer, and melanoma. In another embodiment, the disease to be treated is a haematological cancer, such as a non-Hodgkin's lymphoma, multiple myeloma, chronic lymphocytic leukaemia (CLL), chronic myeloid leukaemia (CML), acute myeloid leukaemia (AML) and acute lymphoblastic leukaemia (ALL). In a more preferred embodiment of the invention, the disease to be treated is AML. In a particularly preferred embodiment, the disease to be treated is a malignant solid tumor.

In another preferred embodiment of the present invention, the tumour to be treated overexpresses CD203a. In an even more preferred embodiment, the tumour to be treated overexpresses both CD203a and CD73.

In a preferred embodiment, the disease to be treated is a chronic infection. The infection can be caused by a viral, bacterial or eukaryotic pathogen. Chronic viral infections to be treated by the antigen-binding polypeptides of the present invention include infections with a human immunodeficiency virus (HIV), infections with a hepatitis C virus (HCV) and infections with a herpes virus. Chronic bacterial infections to be treated by the antigen-binding polypeptides of the present invention include infections with *Haemophilus influenzae, Pseudomonas aeruginosa, Moraxella catarrhalis, Streptococcus pneumoniae* and *Staphylococcus aureus.* Chronic eukaryotic infections to be treated by the antigen-binding polypeptides of the present invention include amoebic infections, such as an infection with *Entamoeba histolytica.*

The therapeutic effect of the polypeptides may be provided by binding of the antigen-binding polypeptide to CD203a and a subsequent modulation, such as a reduction or enhancement, of the enzymatic activity of CD203a on the expressing cells. This means that the binding of the antigen-binding polypeptides of the invention preferably modulates, e.g., reduces or enhances, the enzymatic activity of CD203a to about 80%, 70%, 60%, 50%, 40%, 30%, 20%, 10% or less of the activity of the enzyme measured in the absence of the respective antigen-binding polypeptide.

The therapeutic effect of the polypeptides may also be provided, at least in part, by conjugation to inhibitors of CD203a such as small molecule inhibitors. Effective inhibitors of CD203a have been described in the literature and include, for example, polyoxometalate such as PSB-POM141 (Lee et al. 2015), sulfopolysaccharides from brown and red sea algae, such as fucoidans (Lopez et al. 2021), adenine-N9-(methoxy)ethyl-β-bisphosphonate (Nassir et al. 2019), heparins and its derivatives (Lopez et al., 2023), adenosine 5'-α,β-methylene-γ-thiotriphosphate (Nadel et al. 2014). Further inhibitors include (i) α,β-methylene analogs, (ii) β,γ-methylene analogs, (iii) 2-methylthio-adenine derivatives, (iv) dialdehyde derivatives, (v) diadenosine derivatives and (vi) 2'(3')-*O*-benzoylbenzoyl derivatives (Lee & Müller 2017). Thus, the invention also relates to a conjugate comprising or consisting of CD203a and any of the inhibitors mentioned above.

The therapeutic effect of the polypeptides may also be provided, at least in part, by using the CD203a-specific antigen-binding polypeptides in combination with other antigen-binding polypeptides which are specific for other proteins or non-protein structures expressed on the surface of CD203a-expressing cells. The binding of CD203a-specific and other specific polypeptides to the cell may lead to a particularly efficient therapeutic effect, e.g. reduction of CD203a expression or activity. For example, the CD203a-specific antigen-binding polypeptides may be used in combination with small molecules or antigen-binding polypeptides against targets that induce internalization and/or degradation of CD203a, such as E3 Ligases, MGL1, CI-M6PR, ASGPR, IGF2R, CXCR7, SR-A, Sortillin, TfR1.

The therapeutic effect of the polypeptides may also be provided, at least in part, by reducing soluble CD203a from biological liquids such as plasma or from extracellular vesicles, e.g. by redirecting CD203 to clearance mechanisms such as internalization and degradation by other cells.

The therapeutic effect of the polypeptides may also be provided, at least in part, by killing CD203a-expressing cells, in particular, CD203a-expressing tumour cells. For example, the killing of cells may result from antibody-dependent cell-mediated cytotoxicity (ADCC), complement-mediated cytotoxicity (CDC) and/or apoptotic processes which are induced by binding of the polypeptides of the invention to CD203a. Alternatively, the polypeptides of the invention may be conjugated to one or more cytotoxic agents that result in cell death. Alternatively, the polypeptides of the invention may be used to generate chimeric antigen receptors to generate cells that kill CD203a expressing cells.

Preferably, binding of the antigen-binding polypeptide of the invention to CD203a leads to the death of at least 10%, 20%, 30%, 40%, 50%, 60%, 70%, 80% or even 90% or more of the CD203a-expressing tumour cells of a population.

The CD203a-specific antigen-binding polypeptides disclosed herein may be used in combination with chemotherapy. Specifically, the polypeptides may be used in combination with one or more commonly known chemotherapeutic agents. Chemotherapeutic or cytotoxic agents which is suitable for being co-administered with the polypeptides of the present inventions include, but are not limited to, actinomycin, amsacrine, aminopterin, anthracycline, 5-azacytidine, 6-azauridine, azathioprine, bendamustine, biricodar, bleomycin, bortezomib, bryostatin, busulfan, calyculin, capecitabine, carmofur, carboplatin, chlorambucil, cisplatin, cladribine, clofarabine, cytarabine, dacarbazine, dasatinib, daunorubicin, decitabine, discodermolide, docetaxel, doxorubicin, doxifluridine, epirubicin, eribulin, estramustine, etoposide, exatecan, floxuridine, fludarabine, fluorouracil, 5-FU, fotemustine, gemcitabine, hydroxyurea, idarubicin, ifosfamide, imiquimod, irinotecan, irofulven, ixabepilone, lapatinib, lenalidomide, lomustine, lurtotecan, mafosfamide, masoprocol, mechlorethamine, melphalan, mercaptopurine, mitomycin, mitoxantrone, nelarabine, nilotinib, oxaliplatin, oxazaphosphorine, paclitaxel, pentostatin, pixantrone, plicamycin, procarbazine, raltitrexed, rebeccamycin, rubitecan, salinosporamide A, satraplatin, swainsonine, tariquidar, taxane, tegafur-uracil, temozolomide, testolactone, thiotepa, thioguanine, topotecan, trabectedin, tretinoin, triplatin, tetranitrate, and derivatives, tautomers and pharmaceutically active salts of the above compounds.

The CD203a-specific antigen-binding polypeptides disclosed herein may be used in combination with radiotherapy. As used herein, radiation therapy or radiotherapy refers to the use of ionizing radiation for the targeted destruction of cancer cells or tissues. Radiation therapy includes various techniques for irradiating cancer tissue in a subject with high-energy radiation to destroy the cancer tissue. In the context of the invention, radiation therapy can include photon-based radiation, such as x-rays and gamma rays, and/or particle radiation, such as electron, carbon or proton beams. Preferably, radiation is performed as an external beam radiation therapy. The dose of radiation to be used in combination with the CD203a-specific antigen-binding polypeptides of the invention will depend on the type of tumour to be treated and will normally be in a range of 20-75 Gy, preferably 30-60 Gy and more preferably 45-60 Gy. In a preferred aspect, the daily dose of radiation that is administered is 1.5-1.8 Gy for a child or adolescent and 2.0-2.5 Gy for an adult. Radiation therapy can be applied either before, simultaneously with, or after administration of the CD203a-specific antigen-binding polypeptide.

In one embodiment, the CD203a-specific antigen-binding polypeptides described above are used in combination with other antigen-binding polypeptides which are specific for other structures expressed on the surface of CD203a-expressing cells. The binding of CD203a-specific and other specific polypeptides to the cell may lead to a particularly efficient therapeutic effect, e.g. reduction of tumour cells. For example, the CD203a-specific antigen-binding polypeptides may be used in combination with CD73-specific and/or CD203a-specific antigen-binding polypeptides, such as VHHs which are directed against CD73. Further, the CD203a-specific antigen-binding polypeptides may be used in combination with other compounds that block or target other tumour markers, such as CD38, CD39, CD138, BCMA, CD229 to induce ADCC and/or CDC.

Preferably, the antigen-binding polypeptide of the present invention will have a size that results in physicochemical properties suitable for such therapeutic application, e.g. good solubility and tissue penetration, thermal stability and similar properties. Preferably, the antigen-binding polypeptide of the present invention used for therapeutic treatment of a patient will have a size of 80-250 amino acids, wherein a size of 90-200 amino acids, 90-180 amino acids, 90-150 amino acids, 100-140 amino acids and 100-130 amino acids is preferred.

In a further aspect, the invention provides a method for treating a disease or disorder which is characterized by a pathologically enhanced level of CD203a activity. The treatment comprises administering one or more of the polypeptides of the invention to a patient which suffers from one of the diseases mentioned above, in particular cancer or a chronic disease. Such therapeutic treatment includes both treatment of an acute disease state and prophylactic treatment to prevent disease.

The CD203a-binding polypeptide will preferably be administered in the form of a pharmaceutical composition. Accordingly, the invention also provides a pharmaceutical composition comprising an antigen-binding polypeptide of the present invention. The antigen-binding polypeptide is thereby administered to the patient in a therapeutically effective amount, i.e. in an amount sufficient to significantly modulate, such as reduce or enhance, and preferably reduce, the effect mediated by CD203a in the context of the disease and/or the number of CD203a-expressing cells. In a preferred embodiment of the invention, the antigen-binding polypeptide is administered in an amount that results in a significant modulation, such as reduction or enhancement, and preferably reduction, of the CD203a activity in the patient. In another preferred embodiment of the invention, the antigen-binding polypeptide is administered in an amount that results in a complete reduction of CD203a-expressing cells. The reduction of CD203a activity or CD203a-expressing cells may be local, e.g. in a tumour. The reduction of cells will preferably give rise to an improvement of one or more symptoms of the disease.

In another preferred embodiment of the invention, the CD203a-binding polypeptide is enhancing the activity of CD203a and is thereby suitable for being administered to a patient suffering from a partial ENPP1 deficiency.

In yet another aspect, the present invention relates to the antigen-binding polypeptides of the present invention for use in a method of enhancing an immune response in a subject in need thereof. In yet another aspect, the present invention relates to the antigen-binding polypeptides of the present invention for use in a method of reversing immunosuppression in a subject in need thereof.

The exact amount of polypeptide that needs to be administered to achieve a therapeutic effect depends on several parameters. Factors relevant to the determination of the amount of polypeptide to be administered include, for example, the route of administration of the polypeptide, the size of the polypeptide in question, the nature of the cytotoxic residue (if any), the nature and severity of the disease, the medical history of the patient to be treated, and the age, weight, height and health status of the patient to be treated. A therapeutically effective amount of the antigen-binding polypeptide can be readily determined by one skilled in the art based on the common knowledge and the present disclosure.

The polypeptide is preferably administered to a subject in an amount sufficient to achieve a plasma concentration of from about 0.05 µg/ml to about 150 µg/ml, preferably from about 0.1 to about 50 µg/ml, more preferably from about 1 µg/ml to about 20 µg/ml, and typically from about 1 µg/ml to about 10 µg/ml, e.g. 5 µg/ml. The weekly dose may range from about 1 mg to about 500 mg of polypeptide per m² of body surface area of the patient. The weekly dose of the polypeptide may range from about 10-300 mg/m², preferably from about 100-200 mg/m², such as 150 mg/m². Depending on the size of the polypeptide, the amount of polypeptide to be administered can be adjusted.

The antigen-binding polypeptide of the present invention may be formulated for various modes of administration, e.g. oral administration as a tablet, capsule, powder, liquid or the like. However, it is preferred that the antigen-binding polypeptide is administered parenterally by intravenous injection or intravenous infusion. For example, administration may be by intravenous infusion, e.g. within 60 minutes, within 30 minutes, or within 15 minutes. It is further preferred that the antigen-binding polypeptide is administered locally by injection during surgery. Compositions suitable for administration by injection and/or infusion typically comprise solutions and dispersions, and powders from which such solutions and dispersions can be prepared. Such compositions will comprise the immunologically active polypeptide and at least one suitable pharmaceutically acceptable carrier. Suitable pharmaceutically acceptable carriers for intravenous administration include bacteriostatic water, Ringer's solution, physiological saline, phosphate buffered saline (PBS) and Cremophor EL^{™}. Sterile compositions for injection and/or infusion can be prepared by introducing the polypeptide in the required amount in a suitable carrier and then filtering it sterilely using a filter. Compositions for administration by injection or infusion should remain stable under storage conditions for a prolonged period after their preparation. The compositions may contain a preservative for this purpose. Suitable preservatives include chlorobutanol, phenol, ascorbic acid and thiomersal.

In one embodiment, the antigen-binding polypeptide of the present invention is formulated with carriers that protect the polypeptide from being excreted too rapidly from the body (sustained release formulations). Such formulations may include biocompatible polymers, such as ethylene vinyl acetate, polyanhydrides, polyglycolic acids, collagen, polyorthoesters and polylactic acids. Processes for the preparation of sustained-release formulations are sufficiently known in the prior art. These formulations may be provided in the form of semi-permeable films of hydrophobic polymers, e.g. in the form of microcapsules. The production of corresponding dosage forms as well as suitable excipients is described, for example, in Remington (2005).

In order to increase the therapeutic efficacy of the polypeptides according to the invention, it may be advantageous not only to block the action of CD203a on the surface of CD203a-expressing cells, but also to kill these cells. This can be achieved, for example, by conjugating one or more cytotoxic agents to the antigen-binding polypeptides described herein.

The invention thus also provides a conjugate comprising as a first component an antigen-binding polypeptide as described above, preferably a polypeptide comprising one or more of the CDR regions listed in SEQ ID NO:1-48 and SEQ ID NO:49-129 or modifications thereof as described above, or a polypeptide comprising one of the sequences listed in SEQ ID NO: 130-145 and SEQ ID NO: 146-172 or immunologically active fragments or variants thereof as described above, and as a further component one or more cytotoxic residues. The coupling of the CD203a-specific polypeptides to the cytotoxic agent can be carried out according to procedures customary in the state of the art.

The invention further comprises a viral vector comprising an antigen-binding polypeptide of the present invention. The antigen-binding polypeptides of the present invention are particularly suitable for the use of viral vectors in gene therapy. For example, an antigen-binding polypeptide can be inserted into a protein displayed on the outer surface of a viral vector. Such modification of a viral vector can enhance overall transduction efficacy as the antigen-binding polypeptide redirects the viral vector to its target cells. In a preferred embodiment, the viral vector is an adeno-associated virus (AAV) vector. AAV are commonly used as viral vectors in the arts. However, a limiting factor in their application is their broad tropisms, i.e., parallel infection of several cell types. An AAV vector displaying an antigen-binding polypeptide of the present invention can be used to specifically transduce cells expressing the CD203a protein. In a particularly preferred embodiment, the antigen-binding polypeptide comprises the CDR sequences shown in SEQ ID NO:1-48 and SEQ ID NO:49-129. The antigen-binding polypeptide may be inserted into a capsid protein of the AAV, more preferably, into the VP1 protein of the AAV.

The antigen-binding polypeptide of the present invention can be used in a clinical setting, e.g. for therapeutic or diagnostic purposes, or for research purposes. In one embodiment, the antigen-binding polypeptide of the present invention can be used in immunological and biochemical assays, including microscopy and flow cytometry of living or mildly fixed cells, immunoprecipitation and affinity purification of a target protein, ELISA of biological samples containing antigens, co-crystallization with a target antigen for 3D structure analyses, cryo-electron microscopy analyses of a target protein, and blockade or potentiation of the function of a target protein, such as its enzymatic or channel activity or its binding to specific ligands. In a particular preferred embodiment, the antigen-binding polypeptides of the present invention are used in the form of chimeric antibodies, chimeric antigen-receptors (CARs), bispecific T cell engagers (BiTEs), bispecific killer cell engagers (BiKEs) and nanobody-displaying adeno-associated viruses (AAVs). The above forms of the antigen-binding polypeptides of the present invention may also be constructed as being specific for one, two or multiple antigens and are thus mono-, bi- or multispecific.

Finally, the present invention also provides a kit for the detection of CD203a and CD203a-expressing cells, the kit comprising an antigen-binding polypeptide as described above. The kit may further comprise other reagents and means necessary for the detection, e.g. reagents for visualising a label.

### BRIEF DESCRIPTION OF THE FIGURES

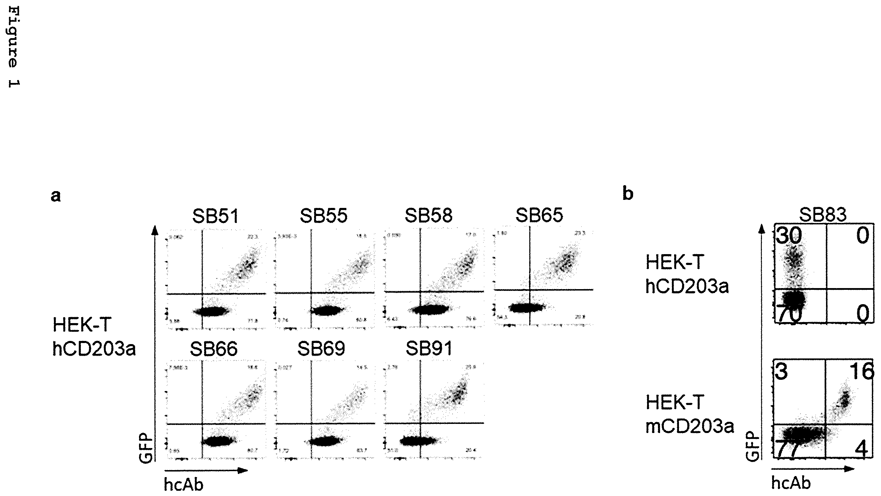

**Figure 1** shows the results of the analysis of hcAbs binding to CD203a-transfected HEK293 cells by flow cytometry. HEK293 cells were co-transfected with expression vectors for GFP and either human CD203a (a) or mouse CD203a (b). After transfection, cells were incubated with HEK-6E cell supernatants containing the indicated recombinant nanobody-rabbit IgG hcAbs. Bound antibodies were detected with PE-conjugated donkey anti-rabbit IgG secondary antibody.

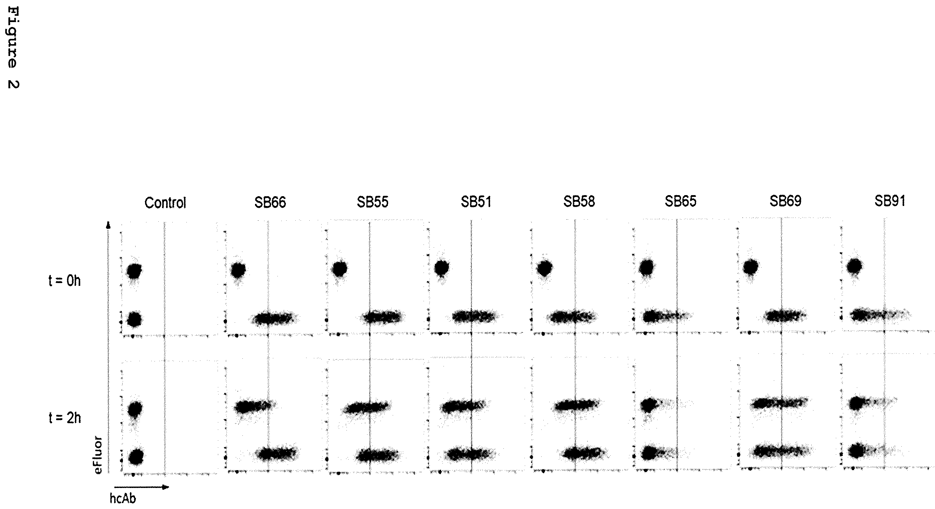

**Figure 2** shows the results of the analysis of the dissociation rate of seven different recombinant nanobody-rabbit IgG hcAbs by flow cytometry. Human CD203a-transfected HEK293 cells were either labelled with eFluor or one of the analysed hcAbs and incubated together for 2h. Bound antibodies were detected with PE-conjugated donkey anti-rabbit IgG secondary antibody.

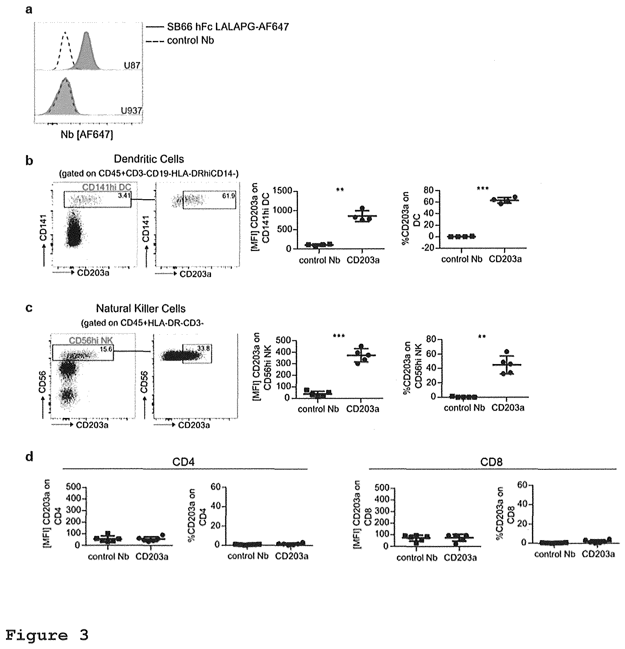

**Figure 3** shows the results of the analysis of binding capacity of the recombinant SB66-hcAbs (either rabbit IgG or the LALAPG mutant of human) to native CD203a present on cancer cell lines (a) and on primary human PBMCs (b) by flow cytometry. For detection, SB66-hcAbs were conjugated with Alexa-Fluor 647 (AF647).

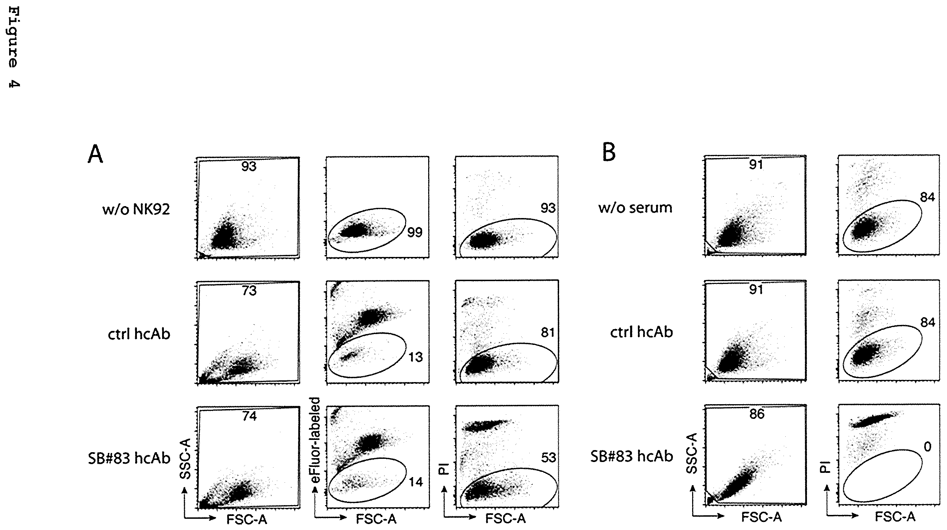

**Figure 4** shows the results of the antibody-dependent cellular cytotoxicity (ADCC) assay and of the complement-dependent cytotoxicity (CDC) assay. MOPC-315 or MOPC-315 preincubated with control hcAb (ctrl hcAb) or SB83-mIgG2a (SB83-hcAb) were incubated with eFluor450-labeled NK92 expressing murine CD16 (a) or with guinea pig serum (b).

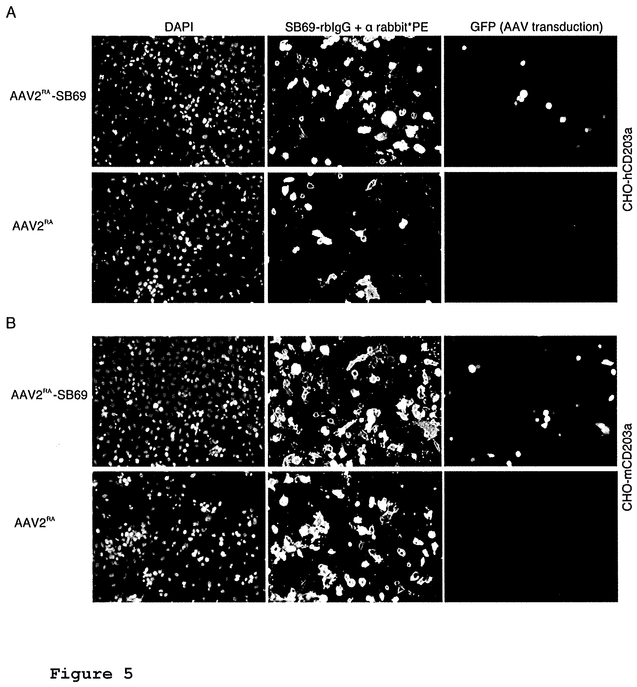

**Figure 5** shows the results of the transduction of CD203a-transfected cells with AAV displaying CD203a-specific nanobody SB69. Human (a) or mouse (b) CD203a expressing CHO cells were incubated with HEK cell supernatant containing GFP-encoding AAV2^{RA} displaying nanobody SB69 (AAV2^{RA}-SB69) or AAV2^{RA} without nanobody. Cell nuclei were stained with DAPI and human/mouse CD203a-transfected cells were stained with SB69-rabbit IgG and secondary PE-conjugated rabbit-specific antibodies. GFP expression was analysed by immune fluorescence microscopy and reflects the level of AAV transduction.

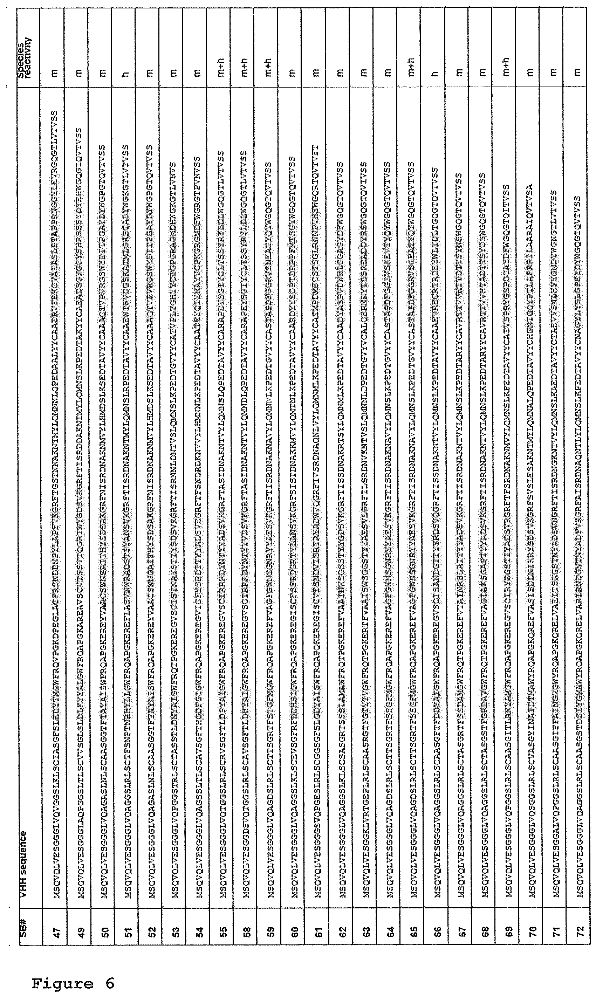

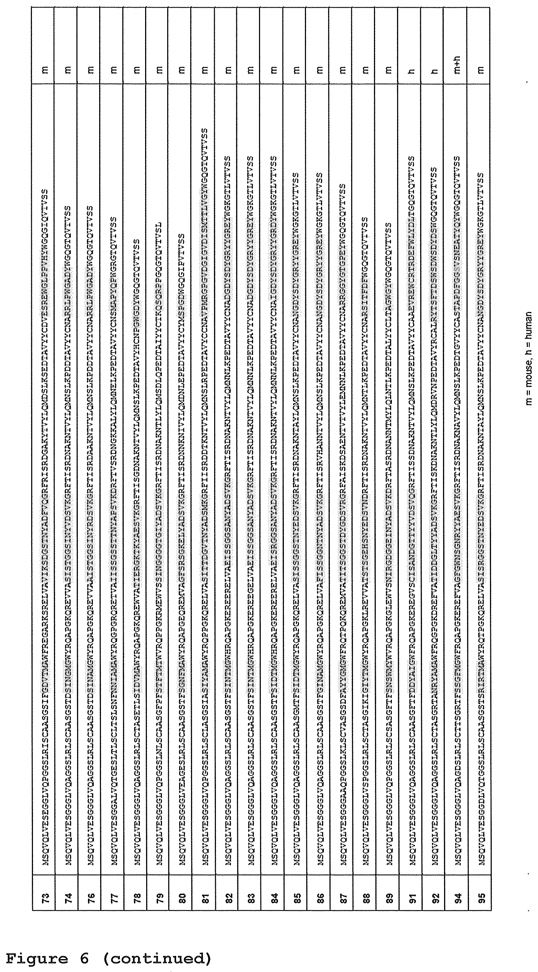

**Figure 6** shows the amino acid sequences of the CD203a-specific VHHs identified in the below working example.

### EXAMPLES

The present invention is further described in more detail by the following examples which are only provided for illustrating the invention and which are not to be construed as limiting the scope of the invention. The following material and methods were used in the Examples.

### 1. Immunizations

1.1 Eucaryotic expression vectors for human CD203a/ENPP1 (gene ID 5167) and mouse CD203a (gene ID 18605) were cloned into pCMV-SPORT6. The ectodomain of CD203a was expressed as a recombinant protein with a C-terminal His6x tag in transiently transfected HEK-cells and purified by immobilized metal affinity chromatography.

1.2 Two alpacas (designated SAL010 and SAL0011) were immunized by a DNA-prime protein boost strategy as described previously (see Koch-Nolte et al. 2007). The humoral immune response was monitored in serially diluted serum on CHO cells transfected with the same plasmids used for immunization by IFM in 96 well microtiter plates on EVOS inverted fluorescence microscope. Animals were bled 7 days after the 3^{rd} or 4^{th} boost.

### 2. Library construction

2.1 Mononuclear cells were isolated from 120 ml of blood by Ficoll-Paque^{™} (GE Healthcare, Chalfont St Giles, UK) gradient centrifugation. RNA purified from these cells by in-nuPREP RNA Mini Kit (Analytic Jena, Jena, Germany) was subjected to cDNA synthesis with alpaca CH2-specific primers. The nanobody coding region was amplified by PCR with degenerate nanobody-specific primers.

### 3. Production of nanobodies and hcAbs

3.1 The coding region of selected nanobodies (Nbs) was cloned using NcoI and NotI into the pCSE2.5 vector (Jäger et al. 2013) upstream of either a myc-C-hexahistidin tag or the hinge and Fc-domains of rabbit IgG, mouse IgG2c or human IgG1 (Eden et al. 2018). Recombinant myc-his tagged Nbs and rabbit IgG, mouse IgG2c, or human IgG1 heavy chain antibodies (hcAbs) were expressed in transiently transfected HEK-6E cells cultivated in serum-free medium. Six days post transfection, supernatants were harvested and cleared by centrifugation.

3.2 Nbs and hcAbs in cell supernatants were quantified by SDS-PAGE and Coomassie staining relative to marker proteins of known quantities: 10 µl samples of the supernatant were size fractionated side by side with standard proteins (albumin 4 µg, IgH 2 µg, IgL 1 µg, lysozyme 0.4 µg; albumin 1 µg, IgH 0.5 µg, IgL 0.25µg, lysozyme 0.1 µg). Yields of recombinant Nbs and hcAbs typically ranged from 0.5-3 µg/10µl.

3.3 Myc-His tagged Nbs were purified by immobilized metal affinity chromatography using Ni-NTA agarose (Sigma, St Louis, MO). Heavy chain antibodies proteins were purified by affinity chromatography using protein A-sepharose (GE-Healthcare).

### 4. Flow cytometry

4.1 For binding analyses of CD203a-specific hcAbs to CD203a-transfected cells, HEK293 cells were transiently co-transfected with expression vectors encoding GFP and either human or mouse CD203a. HEK293 cells were incubated for 30 min with HEK-6E cell supernatants containing rabbit IgG hcAbs (diluted 1:10 in PBS/0.1% BSA). Following washing with PBS/0.1% BSA, bound antibodies were detected with PE-conjugated donkey anti-rabbit IgG polyclonal antibody (711-116-152 Dianova, Hamburg).

4.2 For dissociation analyses, two separate aliquots of human CD203a-transfected cells were incubated either with Cell Proliferation Dye eFluor 450 (eBioscience) or with hcAbs for 20 min at RT. Cells were washed four times, mixed at a 1:1 ratio and further incubated at RT for 120 min before staining of bound antibodies with PE-conjugated donkey anti-rabbit IgG polyclonal antibody. The dissociation of hcAbs from the target cells and association with the eFluor 450 labelled cells was analysed by flow cytometry using the FlowJo software (Treestar).

### Example 1: Induction of CD203a-specific hcAbs

Two alpacas were immunized with CD203a for the induction of hcAbs. In particular, alpacas SAL010 and SAL011 were immunized and boosted with CD203a expression vectors by ballistic immunization essentially as described previously (Koch-Nolte et al 2007). Each alpaca received four DNA immunizations with administration intervals of two weeks. Each dose consisted of 12 shots of plasmid-conjugated gold particles (1 µg of DNA conjugated onto 0.5 mg gold particles per shot) applied with a pressure setting at 600 psi into the skin. Three weeks after the final genetic immunization, a single boost with 2 x 10⁷ CD203a-transfected HEK293 cells was given. At regular intervals, blood samples were collected to monitor the induction of the humoral immune response over time. For the isolation of B-cells, blood was collected from these animals 7 days after the fourth DNA immunization and 7 days after the cell boost. Peripheral blood mononuclear cells were prepared from blood samples using Ficoll-Paque^{™} (GE Healthcare, Chalfont St Giles, UK) gradient centrifugation. The induction of a humoral immune response and reactivity of the immune sera were confirmed by IFM.

### Example 2: Selection of VHHs

CD203a-specific VHH from the immune libraries was performed using IFM-based screening. Plasmid DNA was isolated from single colonies and subjected to sequence analyses using pCSE-specific forward and reverse primers. The sequences of the CD203a-specific VHHs are provided in Figure 6.

### Example 3: Recombinant expression of VHH in HEK-6E cells

Representative VHHs of the above identified VHHs were expressed as myc-his tagged Nbs and as hcAbs comprising a rabbit IgG, mouse IgG2c or human IgG1 in HEK-6E cells. The myc-his tagged Nbs were purified by immobilized metal affinity chromatography using Ni-NTA agarose (Sigma, St Louis, MO). Heavy chain antibodies were purified by affinity chromatography using protein A-sepharose (GE-Helathcare). SDS-PAGE analysis confirmed the purity, integrity and concentration of the purified hcAbs and VHHs (not shown).

### Example 4: Analysis of hcAbs binding to CD203a-transfected HEK293 cells

The binding specificities of purified recombinant nanobody-rabbit IgG hcAbs were determined by flow cytometry using HEK293 cells transiently co-transfected with expression vectors for GFP and either human CD203a or mouse CD203a. The cells were incubated with the hcAb containing HEK-6E cell supernatants for 30 min. After washing, cells were incubated with PE-conjugated donkey anti-rabbit IgG polyclonal antibody (711-116-152 Dianova, Hamburg). The cells were resuspended in PBS and analysed by flow cytometry. Data evaluation was conducted using the FlowJo software (Treestar, Stanford, US).

Results: The results are shown in Figure 1. It can be seen that the analysed hcAbs showed specific binding to human CD203a transfected HEK cells (a) or mouse CD203a transfected HEK cells (b).

### Example 5: Analysis of the dissociation rate of CD203-specific hcAbs

The dissociation rate of the recombinant nanobody-rabbit IgG hcAbs were determined by flow cytometry using human CD203a-transfected HEK293 cells. The cells were incubated with either eFluor or with one of the analysed anti-human CD203a hcAbs (see Example 4). After washing, cells incubated with eFluor and cells incubated with one of the analysed hcAbs were combined. Cell bound hcAbs were detected with PE-conjugated donkey anti-rabbit IgG polyclonal antibody at time point zero and after 2 hours.

Results: The results are shown in Figure 2. Antibody dissociation and reassociation results in a decrease of PE signal intensity in the eFluor-negative population and increase of PE signal intensity in the eFluor-positive population. It can be seen that from the hcAbs tested in Example 4, SB66 has the highest binding capacity and lowest dissociation capacity.

### Example 6: Analysis of hcAbs binding to native CD203a on cancer cell lines and on primary human PBMCs by flow cytometry

Purified anti-CD203a hcAbs SB66 were analysed for binding to native CD203a on the cell surface of cancer cell lines and human peripheral blood immune cells (PBMCs) subsets by flow cytometry. The anti-CD203a hcAbs SB66 (either rabbit IgG or the LALAPG mutant of human) were conjugated with Alexa-Fluor 647 (AF647). For analysis of CD203a binding on cancer cell lines, U87 human glioblastoma cells and U937 histocytic lymphoma cells were chosen and incubated with AF647-SB66-hcAB (a). For analysis of CD203a binding on primary human PBMCs, purified cells were incubated for 30 min at RT with 10 µl of diluted AF647-conjugated SB66 rabbit IgG or AF647-conjugated SB66 human IgG LALAPG. AF647-conjugated rabbit IgG hcAb binding to murine ARTC2.2 was used for negative controls. Cells were co-stained with a standard panel of fluorescently labelled antibodies against human immune cell markers. Gating was performed on different immune cell populations (b-d).

Results: The results are shown in Figure 3. The results show that hcAb SB66 can be used to stain CD203a on cancer cell lines and primary human PBMCs. In particular, use of hcAb SB66 identified specific immune cell subsets with significant expression of CD203a. By contrast, other immune cell subsets were shown not to express CD203a.

### Example 7: Analysis of SB83-hcAb-induced antibody-dependent cellular cytotoxicity (ADCC) and complement-dependent cytotoxicity (CDC)

Cells of the CD203a-expressing murine myeloma cell line MOPC 315 (ATCC TIB-23) were preincubated for 15 min with a saturating amount of SB83 mouse IgG2a (SB83-hcAb). For antibody-dependent cellular cytotoxicity (ADCC) assays, eFluor450-labeled NK92 cells transfected with murine CD16 were added with an effector to target cell ratio of 3:1. For complement-dependent cytotoxicity (CDC) assays, 25% of guinea pig serum was added as a source of complement. Cells were incubated at 37 °C and 5% CO₂ for 3 h (ADCC) or 2 h (CDC). Gating was performed on unlabelled cells without debris and live cells were identified via propidium iodide (PI) staining (1:500).

Results: The results are shown in Figure 4. The results show that CD203a-specific hcAbs induce ADCC and CDC against CD203a-expressing myeloma cell lines.

### Example 8: Analysis of CD203a-specific nanobody SB69-induced Adeno-associated viruses (AAV) transduction of CHO cells

For transduction analysis, CHO cells were transiently transfected with expression vectors encoding either human or mouse CD203a. 24 h post seeding, cells were incubated with 10 µL of HEK cell supernatant containing GFP-labelled AAV^{RA}-SB69 or GFP-labelled AAV^{RA}. Production of AAV was carried out as described previously (see Eichhoff et al. 2019, Nolte et al. 2019). The serotype 2 variant mutant AAV^{RA} is characterized by two arginine substitutions to alanine inhibiting binding to the natural attachment factor of the virus. AAV^{RA}-SB69 is further characterized by a CD203a-specific nanobody SB69 insertion into the VP1 capsid protein. After 72 h of incubation, AVV-containing HEK cell supernatants were harvested and used for CHO cell transduction experiments.

For immune fluorescence microscopy analysis, CHO cells were fixed with 2% PFA, cell nuclei stained with DAPI and human/mice CD203a-transfected cells stained with 5 µL HEK-6E cell supernatant containing SB69-rabbit IgG followed by a secondary PE-conjugated donkey anti-rabbit IgG polyclonal antibody.

Results: The results are shown in Figure 5. The results show that AAV displaying the human (a) or mouse (b) CD203a-crossreactive nanobody SB69 on the viral capsid specifically transduce CHO cells transiently transfected with human or mouse CD203a.

### LITERATURE

1. Eden et al. 2018. Front Immunol. 8:1989. PMID: 29410663
2. Eichhoff et al. 2019. Mol Ther Methods Clin Div. 15: 211-220. PMID: 31687421
3. Jäger et al. 2013. BMC Biotechnol. 13:52. PMID: 23802841
4. Koch-Nolte et al. 2007. FASEB J. 21:3490-8. PMID: 17575259
5. Lee et al. 2015, Biochemical Pharmacology, 93:2, 171-181
6. Lee & Müller 2017, Medchemcomm, 8:5, 823-840.
7. Lopez et al. 2021, Mar Drugs, 19:2, 51
8. Lopez et al. 2023, Front Immunol, 14:1173634
9. Nadel et al. 2014, J Med Chem 57:11, 4677-91
10. Nassir et al. 2019, Purinergic Signal 15:2, 247-263
11. Nolte et al. 2019. WO 2019 211 401.
12. Remington: The Science and Practice of Pharmacy, Lippincott Williams & Wilkins; 21. ed (2005)

## Claims

1. Antigen-binding polypeptide comprising the CDR1, CDR2 and CDR3 region of a VHH domain of a camelid heavy chain antibody, **characterised in that** the polypeptide specifically binds to CD203a.

2. Antigen-binding polypeptide according to claim 1, wherein said polypeptide inhibits tumour growth.

3. Antigen-binding polypeptide according to claim 1 or 2, wherein said CD203a is human CD203a.

4. Antigen-binding polypeptide according to any of claim 1-3, **characterized in that** the polypeptide comprises
(a) the CDR sequences shown in SEQ ID NO:1-3,
(b) the CDR sequences shown in SEQ ID NO:4-6;
(c) the CDR sequences shown in SEQ ID NO:7-9;
(d) the CDR sequences shown in SEQ ID NO:10-12;
(e) the CDR sequences shown in SEQ ID NO:13-15;
(f) the CDR sequences shown in SEQ ID NO:16-18;
(g) the CDR sequences shown in SEQ ID NO:19-21;
(h) the CDR sequences shown in SEQ ID NO:22-24;
(i) the CDR sequences shown in SEQ ID NO:25-27;
(j) the CDR sequences shown in SEQ ID NO:28-30;
(k) the CDR sequences shown in SEQ ID NO:31-33;
(l) the CDR sequences shown in SEQ ID NO:34-36;
(m) the CDR sequences shown in SEQ ID NO:37-39;
(n) the CDR sequences shown in SEQ ID NO:40-42;
(o) the CDR sequences shown in SEQ ID NO:43-45;
(p) the CDR sequences shown in SEQ ID NO:46-48;
or CDR sequences which differ from the CDR sequences shown in (a)-(p) in not more than one amino acid per CDR region.

5. Antigen-binding polypeptide according to claim 3, **characterized in that** the polypeptide comprises an amino acid sequence selected from the group consisting of:
(a) the amino acid sequences of SEQ ID NO:130-145; and
(b) an amino acid sequence having at least 80% or more sequence identity to one of the amino acid sequences of SEQ ID NO:130-145.

6. Conjugate comprising an antigen-binding polypeptide according to any one of claims 1-5 coupled to a cytotoxic moiety or small molecule inhibitor of CD203a.

7. Viral vector comprising an antigen-binding polypeptide according to any one of claim 1-5.

8. Viral vector according to claim 7, wherein the viral vector is an adeno-associated virus (AAV) vector.

9. Pharmaceutical composition comprising an antigen-binding polypeptide according to any one of claims 1-5, a conjugate according to claim 6 or a viral vector according to claims 7-8.

10. Antigen-binding polypeptide of any one of claims 1-5, conjugate of claim 6, viral vector of claims 7-8 or pharmaceutical composition of claim 9 for use in a method of treating a disease which is **characterized by** a pathologically enhanced level of CD203a activity.

11. Antigen-binding polypeptide, conjugate, viral vector or pharmaceutical composition for use in a method according to claim 10, **characterised in that** the disease is a hyperproliferative disease.

12. Antigen-binding polypeptide, conjugate, viral vector or pharmaceutical composition for use in a method according to claim 10, **characterized in that** the hyperproliferative disease is a CD203a-expressing solid tumour selected from the group of gastric cancer, colon cancer, bladder cancer, glioma, glioblastoma, renal cancer, ovarian cancer, melanoma, prostate cancer, thyroid cancer, oesophageal cancer, lung cancer, such as non-small cell lung carcinoma (NSCLC), head and neck cancer, brain cancer, such as astrocytic brain tumors, breast cancer, and melanoma.

13. Nucleic acid molecule encoding an antigen-binding polypeptide according to any one of claims 1-5.

14. Expression vector comprising a nucleic acid molecule according to claim 13.

15. Host cell comprising a nucleic acid molecule according to claim 13 or an expression vector according to claim 14.

16. A method for the recombinant production of an antigen-binding polypeptide according to any one of claims 1-5, comprising:
(a) culturing a host cell according to claim 15 under conditions which allow expression of a nucleic acid molecule according to claim 13; and
(b) isolating the antigen-binding polypeptide from the culture.

17. A method for the purification and/or concentration of CD203a and/or CD203a-expressing cells in a biological sample, which comprises
(a) contacting an antigen-binding polypeptide according to any one of claims 1-5 with the sample under conditions that allow the formation of complexes of the polypeptide and CD203a; and
(b) separating the complexes are from the sample.

18. A method for the detection of CD203a and/or CD203a-expressing cells in a biological sample, comprising
(a) contacting an antigen-binding polypeptide according to any one of claims 1-5 with the sample under conditions that allow the formation of complexes of the polypeptide and CD203a; and
(b) detecting the complexes in the sample.

19. A method for the diagnosis of a disease **characterised by** increased CD203a expression comprising
(a) contacting an antigen-binding polypeptide according to any one of claims 1-5 with a biological sample from a patient under conditions that allow the formation of complexes of the polypeptide and CD203a; and
(b) detecting complexes of the polypeptide and CD203a/CD203a-expressing cells;
wherein detection of the complexes in step (b) indicates that the patient has a disease **characterised by** increased CD203a expression.

20. Method according to claim 19, wherein the method comprises the step of comparing the amount of complexes detected in step (b) with an amount of a control.

21. Method according to claim 19 or 20, **characterized in that** the disease is hyperproliferative disease.

22. Method according to claim 21, **characterized in that** the hyperproliferative disease is a CD203a-expressing solid tumour selected from the group of gastric cancer, colon cancer, bladder cancer, glioma, glioblastoma, renal cancer, ovarian cancer, melanoma, prostate cancer, thyroid cancer, oesophageal cancer, lung cancer, such as non-small cell lung carcinoma (NSCLC), head and neck cancer, brain cancer, such as astrocytic brain tumors, breast cancer, and melanoma.

23. Kit for detecting CD203a and/or CD203-expressing cells, **characterised in that** the kit comprises an antigen-binding polypeptide according to any one of claims 1-5.
